# EUROPEAN PATENT SPECIFICATION

(11) **EP 0 424 534 B1**
(45) Date of publication and mention of the grant of the patent: **22.06.1994**
(21) Application number: 89909908.9
(22) Date of filing: 28.04.1989
(51) Int. Cl.: A61K 35/78

(54) **PHARMACEUTICAL PREPARATION FOR TREATMENT OF MASTITIS IN ANIMALS AND HUMANS**
ARZNEIMITTELZUBEREITUNG ZUR BEHANDLUNG VON MASTITIS BEI TIER UND MENSCH
PREPARATION PHARMACEUTIQUE DE TRAITEMENT DE LA MASTITE CHEZ L'ANIMAL ET CHEZ L'HOMME

(43) Date of publication of application: 02.05.1991
(73) Proprietor: DERYABIN, Alexandr Mikhailovich, Moscow, 119034 (RU)
(72) Inventor: DERYABIN, Alexandr Mikhailovich, Moscow, 119034 (SU)
(74) Representative: Wain, Christopher Paul
(86) International application number: SU8900115
(87) International publication number: WO9013305

(56) References cited:
- FR-A- 2 296 426
- FR-A- 2 441 389
- D.K. Chervyakov et al. "Lekarstvennye sredstva v veterinarii", "Kolos", (Moscow), page 435
- M.I. Rabinovich "Lekarstvennye rastenia v veterinarii", 1981, Posselkhozizdat, (Moscow), pages 96-97

## Description

The present invention relates to a medicinal agent composition for treatment of mastitis in animals and humans.

At present, there are many different chemotherapeutic agents used for the treatment of mastitis in animals and humans. The majority of these agents are antibiotics, penicillin and streptomycin being the most commonly applied drugs (81 percent).

A variety of medicinal compositions are currently used for treatment of mastitis in animals, the compositions being based on antibiotics and incorporating an oleaginous base, prednisolone, a foaming agent, and a propellant. However, these agents are not adequately efficient. Treatment of mastitis with antibiotic-based drugs causes side effects and gastrointestinal disorders, and affects an organism's resistance. Also, the meat and milk from affected animals contain residual quantities of medicinal substances used in the treatment of mastitis.

It is also known to treat mastitis using ichthammol (an ammonium salt of sulfo acids of shale oil) (cf. Medicinal agents and biopreparations in furbreeding, A Handbook, by F.G. Nabiev, A.A. Dragumov, and R.G. Rakhmatullin, 1986, Agropromizdat Publishers, Moscow, pp. 110-111; Medicinal agents in veterinary medicine, by D.K. Cherviakov, P.D. Yevdokimov, and A.S. Vishker, 1970, Kolos Publishers, Moscow, p. 300 [in Russian]).

Ichthammol features an antimicrobial, antipyretic, locally anaesthetic and keratoplastic action, its main active principles being sulfur and some aromatic substances. The drug is applied as a 10- to 30- percent ointment or 10-to 20- percent alcoholic solution. Ichthammol ointment is applied to the animal's udder affected by mastitis, along with fats or in a glycerol solution. However, ichthammol ointment is a low-efficacious drug, since it is poorly absorbed by the tissue being treated and does not easily penetrate thereinto, especially into deeply seated layers. The ointment is most commonly applied in combination with some other therapeutic substances.

We have now devised a novel medicinal agent composition having high therapeutic efficacy in the treatment of mastitis, and which is nontoxic and free from any side effects.

According to the present invention, there is provided a medicinal agent composition for treatment of mastitis in animals and humans, said medicinal agent comprising a mixture of a decoction of, and an ammonia-solution infusion of, the following medicinal herbs taken in equal parts by weight:
wild camomile,
pot marigold (flowers),
stinging nettle,
common centaury,
pine buds,
common plantain,
birch buds,
pot marjoram,
garden sage,
garden angelica,
dandelion,
coltsfoot (leaves),
great burnet,
common valerian,
peppermint,
common thyme,
tripartite bur-marigold;
said decoction and said infusion being mixed in a ratio to give a pH value of the mixture not below 7.4.

The invention also includes the use of a medicinal agent composition of the invention in the manufacture of a medicament to treat mastitis in animals and humans. The composition preferably contains an infusion of said herbs in a 25-percent aqueous ammonia solution.

Preferably, the medicinal agent composition of the invention contains the following volume percent ratios:

| | |
|---|---|
| a decoction of said herbs, | 70 to 80; |
| an infusion of said herbs, | 30 to 20. |

The composition of the invention may also contain a mixture of a decoction of, and an infusion of, the following herbs taken in equal parts by weight: small-leaved lime (flowers), bog cudweed, motherwort, common immortelle, celandine poppy, southern blue gum-tree, milfoil (flowers), common Saint-John's wort, senna leaves, fennel (seeds).

The medicinal agent composition of the invention possesses highly efficient action in the treatment of mastitis; its application can result in 98 to 100-percent recovery.

Application of the agent composition improves organoleptic properties of milk and destroys its pathogenic flora in convalescent animals, as well as contributing to prompt tissue regeneration in cases of injuries.

Unlike treatment with antibiotics, which is followed as a rule by partial atrophy of the mammary gland and its reduced function, treatment with the agent of the invention results in enhanced function of the mammary gland and hence an increased amount and a better quality of milk given.

The use of the agent composition of the present invention for treatment of mastitis in cows is conducive to a fast-rate restoration of milk-yield at diaries.

The medicinal agent composition of the invention is a mixture of a decoction of, and an ammonia-solution infusion of, a number of medicinal herbs, said decoction and said infusion being taken in a ratio that provides for the pH value of said mixture not below 7.4.

The following herbs are used as said medicinal herbs for preparation of the aforesaid decoction and infusion: wild camomile (Matricaria chamomilla L.), pot marigold [flowers] (Calendula officinalis L.), stinging nettle (Urtica dioica L.), common centaury (Erythreae centaurium Raf.L.), pine buds (Gemmae Pini), common plantain (Plantago major L.), birch buds (Gemmae Betulae), pot marjoram (Origanum vulgare L.), garden sage (Salvia officinalis L.), garden angelica (Archangelica officinalis Hoffm.), dandelion (Taraxacum officinalis Web.), coltsfoot [leaves] (Tussilago farfara L.), great burnet (Sanguisorba officinalis L.), common valerian (Valeriana officinalis L.), peppermint (Mentha piperita L.), common thyme (Thymus vulgaris L.), tripartite bur-marigold (Bidens tripartita L.).

This set of medicinal herbs is indispensable for preparation of the agent composition. To attain more efficient action of the proposed composition, the following herbs may be added to the aforesaid set: small-leaved lime [flowers] (Tilia cordata Nill), bog cudweed (Gnaphalium uliginosum L.), motherwort (Leonurus cardiaca L.), common immortelle (Helichrysum arenarium Moench), calendine poppy (Chelidonium majus L.), southern blue gum-tree (Eucalyptus globulus Labill.), milfoil [flowers] (Achillea millefolium L.), common Saint-John's-wort (Hypericum perforatum L.), senna leaves (folium Cassia, Cassia acutifolia Del.), fennel [seeds] (Foeniculum vulgare Mill.) A decoction and an infusion can be prepared from said herbs as follows.

On to a mixture of the herbs, taken in equal parts by weight, is poured boiling water (three litres per 100 to 130g of said mixture of the medicinal herbs). In 7 to 8 hours the mixture is subjected to colation. To prepare an infusion, on to a mixture of the aforementioned herbs taken in equal weight parts, is poured ammonia solution (preferably a 25-percent aqueous solution), taking an amount of three litres of such an aqueous ammonia solution per 100 to 130g of said mixture of the medicinal herbs. The mixture is allowed to infuse for 7 to 10 days and then is subjected to colation. The preprepared decoction and infusion are then mixed in such a ratio that the pH of the resultant mixture is not below 7.4. It is expedient to intermix the decoction and infusion in the following ratio (vol.%):

| | |
|---|---|
| decoction, | 70 to 80; |
| infusion, | 30 to 20. |

The finished product thus obtained is essentially a liquid coloured dark cherry-red, having an odour of ammonia and of medicinal herbs.

The medicinal agent composition contains a large number of diverse biologically potent substances. Thus, the plants incorporate materials such as alkaloids, glycosides, tanning agents, saponins, flavonoids, various organic acids, vitamins, glyceride and essential oils and microelements. All these substances, while acting on a living organism integrally cause a physiological effect even when applied in very low amounts. The agent of the invention has been tested experimentally on test animals and clinically on patients.

The biological effect of the proposed agent composition has been studied on laboratory animals.

Experiments were performed using test rats having a mass of 210 to 230g, which were given the agent externally by applying it to the skin as a strip 20mm wide and 100 to 110 mm long running along the spine, in a dose of 10ml for a first group of the animals, 20ml for a second group of the animals, and 30ml for a third group of the animals. The application of the agent composition was repeated many times until the agent dried completely on the area mentioned above. Repeated application of the proposed agent composition to the test animals' skin was carried out at 55 to 60-minute intervals for six hours. The test animals were followed-up for 5 days.

The studies performed demonstrated that the animals to which the agent composition was applied within the initial two or three minutes of the experiment, exhibited unrest, which was expressed in active movement of the animals over the cage and attempts to get rid of the agent by shaking the hair-covering. In all test animals the skin at the area of application of the proposed agent was slightly hyperemic, while skin hyperemia disappeared in 6 to 8 hours after the last application procedure. It was established by morphological and biochemical blood examinations that the concentration of erythrocytes and haemoglobin in the blood of the experimental animals increased by 2.4 to 3.9 and 3.3 to 6.8 percent, respectively, while the blood sugar content increased by 3.7 to 9.3 percent (P > 0.05), the lysozyme activity, by 10.1 to 12.6 percent (P < 0.05), and the glycogen and ascorbic acid content of the liver, by 13.3 to 14.3 percent, respectively.

No changes in the test animals' behaviour were found within the follow-up period; the animals took food and water unobstructedly, they were mobile, no mortality cases occurred in that period of time.

The examinations performed have demonstrated that the medicinal agent composition of the invention is not toxic for animals when applied to the skin.

The composition has been tested on diverse farm animals for treatment of various-etiology mastitis, i.e. serous mastitis, catarrhal, fibrinous, and suppurative-catarrhal mastitis.

The composition was applied to the inflamed area only. Then rubless massage was carried out on the affected area for 5 to 7 minutes until the skin of said area turned pink. The procedure mentioned above was repeated two or three times a day after milking the affected animal dry. Milking was carried out in the course of massage as well. As a result of application of the proposed agent the wounds were cleared of purulent-necrotic debris, whereupon the wounds healed up and edema disappeared in 2 to 5 days. A treatment course lasted from 3 to 15 days depending on the degree of severity of the disease. A control examination carried out in 15 days demonstrated complete destruction of pathogenic microflora in milk and restoration of the milk-yield of the affected animals.

The medicinal agent composition of the invention was also tested clinically on human patients suffering from the following diseases: purulent mastitis, and serous mastitis. The proposed agent was applied to the inflamed area, the nipples exclusive until the skin of that area turned pink or a light tingling sensation appeared in said area. The procedure was repeated two or three times a day, having withdrawn milk from the mammary gland before application of the proposed agent. The patients thus treated felt amelioration within the first days of said procedure, the body temperature normalized, painful sensations alleviated. In the following days painful sensations disappeared completely, mammary engorgement ceased, induration resorbed. The treatment course lasted 3 to 15 days until complete recovery of the patients.

The composition caused neither complications nor side effects, and no contraindications for its use were observed.

To promote understanding of the present invention, some specific Examples of practical trials of the agent composition are now given.

### Example 1

A total of 45 cows affected by mastitis of a different degree of severity were treated with the agent.

As a result of clinical examination of the animals' udders, it was found that the diseased animals suffered from the following forms of mastitis:

| | |
|---|---|
| serous and catarrhal | 20 heads, |
| fibrous | 16 heads, |
| suppurative-catarrhal | 9 heads. |

Treatment of 16 cows affected by the fibrous form and of 9 animals with suppurative-catarrhal mastitis, using conventional therapy failed to provide positive results.

The agent composition of the invention was applied to the udder skin of the affected animals after each milking, whereupon light massage was done for 5 to 7 minutes three times a day.

As a result of the treatment, all the 20 animals of the first group (suffering from serous and catarrhal mastitis) recovered on the 2nd or 5th days, accompanied by complete restoration of the milk-yield.

The animals of the second group with fibrous mastitis (16 heads) recovered on the 8th or 10th day of treatment and their milk-yield restored gradually.

7 animals of the third group suffering from suppurative-catarrhal mastitis, were treated for 15 days, whereupon they were considered clinically sound.

In two cows with the severest chronic form of suppurative-catarrhal mastitis and atrophy of the teats, the inflammatory process was arrested by the 30th day of treatment, whereupon milk secretion appeared. However, milk-yield of those cows was not restored completely. Thus, of 45 diseased animals subjected to treatment with the composition agent of the invention, 43 cows were cured completely and returned to a milk-producing group.

### Example 2

Four cows affected with following forms of the diseases were treated with the composition of the invention serous mastitis of the right anterior lobe; fibrous mastitis of the left anterior and posterior lobes; fibrous mastitis of the left posterior lobe, accompanied by massive induration; serous mastitis of the left anterior and the right anterior lobes, accompanied by bad edema of the udder.

The agent composition of the invention was applied to the affected area and light rubless massage was carried out for 5 to 7 minutes, the specific consumption of the agent being 100ml per animal. The agent was applied three times a day after the cows had been milked dry. The treatment was carried out by the open method, without bandages or dressings. No changes in the routine upkeep and feed ration of the animals were made.

The treatment process of individual animals proceeded as follows.

For the first cow, with a diagnosis of serous mastitis of the right anterior lobe, treatment was started four days after the onset of the disease. The agent was applied to the affected area three times daily after milking. Painful sensations and induration of the udder lobe reduced considerably on the second day of treatment, complete recovery occurred on the third day.

For the second cow, with a diagnosis of fibrinous mastitis of the left anterior and posterior lobes, treatment began on the third day after the onset of the disease. On the second day of treatment with the agent the cow became quiet, the left anterior lobe got soft, while induration in the left anterior lobe persisted but became soft and reduced in size twofold. After a three-day treatment course the cow was considered sound.

For the third cow, with a diagnosis of fibrous mastitis of the left posterior lobe, massive induration on the left side, treatment started three days after the disease had been revealed. After two days of treatment with the agent the cow became quiet, the induration got soft and reduced in size considerably. After a four-day treatment course the induration disappeared fully.

The fourth cow had the diagnosis: serous mastitis of the left anterior and the right anterior lobes, accompanied bad edema of the udder, considerable painfulness and sanious discharge from the affected udder lobes. The cow was agitated and was not amenable to milking.

After a three-day treatment course with the agent composition the udder became soft, the tumour decreased, and the sanious discharge ceased. The cow became quiet and was amenable to milking well.

### Example 3

A total of 48 cows affected with chronic forms of mastitis were taken from a herd of dairy cows made up of 800 heads for testing the medicinal agent composition.

Clinical examination established that the diseased animals suffered from the following forms of mastitis:

| | |
|---|---|
| serous | 4 heads |
| serous-catarrhal | 18 heads |
| catarrhal | 21 heads |
| fibrinous | 1 head |
| suppurative-catarrhal | 3 heads |
| hemorrhagic | 1 head. |

Treatment of 28 animals by conventional methods failed to provide positive results.

The agent composition was applied to the affected area and the latter was subjected to rubless massage for 5 to 7 minutes. The preparation was applied three times a day after the cows had been stripped of milk. No changes in the routine upkeep and feed ration of the animals were made. The treatment course lasted 9 days. As a result of the treatment all the 48 cows were considered to get sound.

### Example 4

A total of 31 animals affected by the various forms of mastitis were taken out of a herd of 967 dairy cows.

Clinical examination revealed that the diseased animals suffered from the following forms of mastitis:

| | |
|---|---|
| serous and catarrhal | 8 heads |
| fibrous | 7 heads |
| suppurative-catarrhal | 16 heads |

Treatment of 7 animals with fibrous mastitis and 16 animals with suppurative-catarrhal mastitis using traditional methods failed to give positive results.

The agent composition of the invention was applied to the affected area in each of the diseased animals after each milking, followed by light massage for 5 to 7 minutes three times a day.

As a result of the treatment given to the animals of the first group (serous and catarrhal mastitis), the affected animals recovered on the 3rd or 6th day with restoration of the milk-yield, whereas three cows with the severest form of mastitis were treated further.

Thus, 28 animals out of 31 subjected to treatment with the agent, were cured and returned to a milk-producing group.

### Example 5

A total of 64 cows affected by the various forms of mastitis were taken out of a herd of dairy cows. It was established after a clinical examination that the diseased animals suffered from the following forms of mastitis:

| | |
|---|---|
| catarrhal | 39 |
| fibrinous | 9 |
| suppurative-catarrhal | 16. |

Treatment of 18 diseased animals using conventional methods failed to provide positive results.

The treatment course lasted 13 days on average.

The agent composition of the invention was applied to the affected area after the cows had been milked dry, whereupon rubless massage of said area was carried out for 5 to 7 minutes until the latter turned pink.

As a result of the treatment performed, 63 out of 64 treated cows exhibited complete recovery.

### Example 6

A total of 30 cows affected by mastitis were subdivided into two groups. 16 cows of the test group were treated with the agent composition of the invention whereas 14 animals of the control group were given antibiotics.

One of the cows in the control group was sent to a packing house on account of its too low milk-yield.

The test data obtained are tabulated in the table below.

| Groups of cows | Diagnosis | No. of cows | Duration of treatment (days) | No. of cows treated | | |
|---|---|---|---|---|---|---|
| | | | | recovered | improved state | nonrecover ed |
| Test | Catarrhal mastitis | 9 | 2 | 9 | - | - |
| | Serous mastitis | 3 | 5.3 | 3 | - | - |
| | Serous -catarrhal mastitis | 4 | 7 | 3 | 1 | - |
| Control | Catarrhal mastitis | 7 | 7 | 3 | 3 | 1 |
| | Serous-catarrhal mastitis | 6 | 6 | 2 | 2 | 2 |

## Claims

1. A medicinal agent composition for treatment of mastitis in animals and humans, characterized in that it comprises a mixture of a decoction of, and an ammonia-solution infusion of, the following medicinal herbs taken in equal parts by weight:
wild camomile,
pot marigold (flowers),
stinging nettle,
common centaury,
pine buds,
common plantain,
birch buds,
pot marjoram,
garden sage,
garden angelica,
dandelion,
coltsfoot (leaves),
great burnet,
common valerian,
peppermint,
common thyme,
tripartite bur-marigold,
said decoction and said infusion being mixed in a ratio to give a pH value of the mixture not below 7.4.

2. A composition as claimed in Claim 1, characterized in that it contains an infusion of said herbs in a 25-percent aqueous ammonia solution.

3. A composition as claimed in Claim 1 or 2, characterized in that the mixture is in the following volume percent ratios:
| | |
|---|---|
| a decoction of said herbs | 70 to 80, |
| an infusion of said herbs | 30 to 20. |

4. A composition as claimed in Claim 1,2 or 3, characterized in that it contains additionally a mixture of a decoction and an infusion of the following herbs taken in equal parts by weight:
small-leaved lime (flowers)
dog cudweed,
motherwort,
common immortelle,
celandine poppy,
southern blue gum-tree,
milfoil (flowers),
common Saint-John's-wort,
senna leaves,
fennel (seeds).

5. The use of an agent composition as claimed in any of claims 1 to 4 in the manufacture of a medicament to treat mastitis in animals and humans.

## Patentansprüche

1. Therapeutisch verwendbare Mischung zur Behandlung von Mastitis bei Tier und Mensch, dadurch **gekennzeichnet,** daß sie ein Gemisch aus einem Dekokt und einem Auszug der nachfolgenden, in gleichen Gewichtsanteilen verwendeten Heilpflanzen bzw. -kräuter in einer Ammoniaklösung darstellt:
Echte Kamille,
Gartenringelblume (Blüten),
Große Brennessel,
Tausendguldenkraut,
Kieferknospen,
Breitwegerich,
Birkenknospen,
Oregano,
Echter Salbei,
Engelwurz,
Löwenzahn
Gemeiner Huflattich (Blätter),
Großer Wiesenknopf,
Echter Baldrian,
Pfefferminze,
Thymian,
Dreiteiliger Zweizahn,
wobei das Dekokt und der Auszug miteinander in einem solchen Verhältnis gemischt sind, daß der pH-wert des Gemisches nicht unter 7,4 liegt.

2. Mischung nach Anspruch 1, dadurch **gekennzeichnet,** daß sie einen Auszug der Kräuter in 50%-iger wäßriger Ammoniaklösung enthält.

3. Mischung nach Anspruch 1 oder 2, dadurch **gekennzeichnet,** daß sie folgende Volumenprozentverhältnisse aufweist:
| | |
|---|---|
| Ein Dekokt aus den Pflanzen | 70 bis 80, |
| einen Auszug aus den Pflanzen | 30 bis 20. |

4. Mischung nach Anspruch 1, 2 oder 3, dadurch **gekennzeichnet,** daß sie zusätzlich noch ein Gemisch aus einem Dekokt und einem Auszug der folgenden, in gleichen Gewichtsteilen verwendeten Heilpflanzen bzw. -kräuter enthält:
Winterlinde (Blüten),
Sumpfruhrkraut,
Echtes Herzgespann,
Sandstrohblume
Schöllkraut,
Fieberbaum,
Schafgarbe (Blüten),
Gemeines Johanniskraut,
Sennesblätter,
Fenchel (Samen).

5. Verwendung einer therapeutisch wirksamen Mischung nach einem der Ansprüche 1 bis 4 für die Herstellung eines Medikaments zur Behandlung von Mastitis bei Tier und Mensch.

## Revendications

1. Composition d'agents médicinaux pour le traitement de la mastite chez les animaux et chez les humains, caractérisée en ce qu'elle comprend un mélange d'une décoction et d'une infusion faite avec une solution ammonia-cale, des herbes médicinales suivantes prises chacune en poids égal :
matricaire camomille,
souci de culture (fleurs),
ortie,
petite centaurée,
bourgeons de pin,
plantain commun,
bourgeons de bouleau,
marjolaine cultivée,
sauge cultivée
angélique cultivée,
pissenlit,
tussilage (feuilles),
pimprenelle des près,
valériane commune,
menthe poivrée,
thym commun,
bident,
ladite décoction et ladite infusion étant mélangées dans un rapport permettant d'avoir un pH du mélange qui n'est pas inférieur à 7,4.

2. Composition selon la revendication 1, caractérisée en ce qu'elle contient une infusion desdites herbes faite avec une solution aqueuse à 25% d'ammoniac.

3. Composition selon la revendication 1 ou 2, caractérisée en ce que le mélange se fait dans les rapports volumiques suivants :
| | |
|---|---|
| une décoction desdites herbes | 70 à 80 parties, |
| une infusion desdites herbes | 30 à 20 parties. |

4. Composition selon la revendication 1, 2 ou 3, caractérisée en ce qu'elle contient en plus un mélange d'une décoction et d'une infusion des herbes suivantes, prises chacune en poids égal :
tilleul à petites feuilles (fleurs),
gnaphale,
léonure,
immortelle commune,
petite chélidoine,
eucalyptus bleu,
millefeuille (fleurs),
millepertuis,
séné (feuilles),
fenouil (graines).

5. Utilisation d'une composition d'agents comme revendiqué dans l'une quelconque des revendications 1 à 4 dans la fabrication d'un médicament pour traiter la mastite chez la femme et chez des animaux.
